(19) Europäisches Patentamt / European Patent Office / Office européen des brevets

(11) EP 2 931 212 B1

(12) EUROPÄISCHE PATENTSCHRIFT

(45) Veröffentlichungstag und Bekanntmachung des Hinweises auf die Patenterteilung:
**22.02.2017 Patentblatt 2017/08**

(21) Anmeldenummer: **13802301.5**

(22) Anmeldetag: **29.11.2013**

(51) Int Cl.:
***A61K 8/19*** *(2006.01)* ***A61K 8/34*** *(2006.01)*
***A61K 8/41*** *(2006.01)*

(86) Internationale Anmeldenummer:
**PCT/EP2013/075053**

(87) Internationale Veröffentlichungsnummer:
**WO 2014/090597 (19.06.2014 Gazette 2014/25)**

(54) **MITTEL ZUM FÄRBEN UND/ODER AUFHELLEN VON KERATINISCHEN FASERN OHNE AMMONIAKGERUCH**

COMPOSITION TO COLOR OR BLEACH CERATINIC FIBERS WITHOUT AMMONIA SMELL

COMPOSITION POUR COLORER OU BLANCHIR LES CHEVEUX SANS ODEUR AMMONIATIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.12.2012 DE 102012223206**

(43) Veröffentlichungstag der Anmeldung:
**21.10.2015 Patentblatt 2015/43**

(73) Patentinhaber: **Henkel AG & Co. KGaA
40589 Düsseldorf (DE)**

(72) Erfinder:
- **NEUBA, Constanze
41516 Grevenbroich (DE)**
- **JANSSEN, Frank
51103 Köln (DE)**

(56) Entgegenhaltungen:
**WO-A1-2009/043613 WO-A1-2010/015441
WO-A2-2010/066720 WO-A2-2012/079953**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind Mittel zum Färben und/oder Aufhellen von keratinischen Fasern, insbesondere menschlichen Haaren, enthaltend in einem kosmetischen Träger eine spezielle Kombination aus drei Alkalisierungsmitteltypen, mindestens einem nichtionischen Emulgator vom Typ der ethoxylierten Fettalkohole mit einem Ethoxylierungsgrad von 80 bis 120 und mindestens speziellen einem höherkettigen Fettalkohol. Weiterhin ist ein anwendungsbereites Mittel, das durch Vermischen des vorgenannten Mittels mit einer weiteren separaten Komponente enthaltend Wasserstoffperoxid hergestellt wird, Gegenstand der vorliegenden Erfindung.

Zur Bereitstellung farbverändernder kosmetischer Mittel, insbesondere für keratinische Fasern, wie beispielsweise Haare, kennt der Fachmann je nach Anforderung an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Die Oxidationsfärbemittel zeichnen sich durch hervorragende, lang anhaltende Färbeergebnisse aus. Neben der Färbung ist auch das Aufhellen bzw. Blondieren der eigenen Haarfarbe der ganz spezielle Wunsch vieler Verbraucher. Dazu werden die die Faser färbenden natürlichen oder künstlichen Farbstoffe meist oxidativ unter Einsatz von entsprechenden Oxidationsmitteln, wie beispielsweise Wasserstoffperoxid, entfärbt.

Um eine zufrieden stellende Färbe- und Aufhellleistung zu entfalten, benötigen oxidative Färbe- bzw. Aufhellmittel bei der Anwendung in der Regel einen alkalischen pH-Wert, insbesondere bei pH-Werten zwischen 8,5 und 10,5 werden optimale Ergebnisse erzielt.

Zur Einstellung dieser pH-Werte ist bis zum heutigen Zeitpunkt Ammoniak das Alkalisierungsmittel der Wahl. Mit Ammoniak lässt sich nicht nur der für die Farbstoffbildung notwendige pH-Bereich einstellen, sondern Ammoniak sorgt auch in stärkerem Maß als alle anderen bekannten Alkalisierungsmittel für eine Quellung der Haare. Gleichzeitig wirkt Ammoniak als Penetrations- bzw. Penetrationshilfsmittel.

Die mit dem Einsatz von Ammoniak verbundenen anwendungstechnischen Vorteile sind so vielfältig, dass Ammoniak trotz seines unangenehmen, stechenden Geruchs in einer Vielzahl handelsüblicher oxidativer Färbemittel verwendet wird.

Aus der Literatur sind bereits umfangreiche Bestrebungen zur Reduzierung des Ammoniak-Geruchs bekannt. Hierbei bestehen zur Minimierung des Geruchs unterschiedliche Möglichkeiten: Als erste Möglichkeit nennt die Literatur die Variation des Alkalisierungsmittels und damit den teilweisen oder vollständigen Ersatz von Ammoniak durch geruchslose Alternativen.

In der Literatur existiert bereits eine Vielzahl von Rezepturen, welche ein Gemisch von Ammoniak und Monoethanolamin oder ausschließlich Monoethanolamin als Alkalisierungsmittel einsetzen. Die Reduktion des Ammoniak-Gehaltes hat jedoch oft eine schlechtere Penetration der Farbstoffe in das Haar hinein zur Folge, was sich insbesondere in schlechteren Grauabdeckungen und einer schlechteren Waschechtheiten niederschlagen kann.

Obwohl der vollständige oder teilweise Austausch von Ammoniak durch alternative Alkalisierungsmittel sich im Hinblick auf die Geruchsminimierung der Färbe- bzw. Aufhellmittel vorteilhaft auswirkt, ist er im Hinblick auf die Echtheitseigenschaften der mit den Färbe- bzw. Aufhellmitteln erhaltenen Färbungen mit Nachteilen verbunden. Wird ein entsprechender Austausch des Alkalisierungsmittels vorgenommen, müssen die hieraus resultierenden Einbußen hinsichtlich der Färbeleistung durch eine Optimierung der Formulierung kompensiert werden.

Die WO 2006060570 und WO 2006060565 schlagen für die Bereitstellung von oxidativen Färbemitteln mit geringer Geruchsbelastung den Einsatz von Carbonaten oder Carbonatquellen als Alkalisierungsmittel vor. Es ist jedoch ebenfalls literaturbekannt, dass Carbonate in Kombination mit Oxidationsmitteln die Haare in stärkerem Ausmaß schädigen können. Die durch die Carbonate hervorgerufene zusätzliche Schädigung des Haares mag bei Anwendung des Färbemittels auf unbehandeltem bzw. ungeschädigtem Haar wenig problematisch sein, kann sich jedoch bei Personen, die ihr Haar regelmäßig färben bzw. blondieren, zu schweren, kumulativen Schäden aufsummieren. Wird eine stärkere Aufhellung und/oder regelmäßige Färbung gewünscht, stellt der Einsatz von Carbonaten daher ebenfalls keine gangbare Alternative dar.

Eine zweite prinzipielle Möglichkeit zur Reduktion des Ammoniak-Geruchs besteht im Zusatz von speziellen Parfumstoffen, welche den Ammoniak-Geruch überdecken sollen. Dieser Weg wird beispielsweise in der WO 2005/110499 beschritten. Parfumstoffe können jedoch unter den alkalischen Lagerbedingungen instabil sein, so dass die Gefahr besteht, dass die Duftstoffe während der Lagerung abgebaut oder strukturell verändert werden, was sich auch in einer unvorhersehbaren Änderung des Geruchs niederschlägt. Da entsprechende Veränderungen oftmals erst nach mehreren Monaten oder sogar Jahren wahrnehmbar werden, wird der Einsatz von neuen bzw. unbekannten Parfums als problematisch eingestuft. Eine dritte prinzipielle Möglichkeit zur Reduktion des Ammoniakgeruchs besteht in einer Optimierung der Formulierung. Hierbei gilt es, die Träger-Bestandteile der Formulierung so auszuwählen, dass diese einen optimalen Rückhalt des Ammoniaks in der Formulierung gewährleisten und auf diese Weise seinen Geruch minimieren. Es ist jedoch ebenfalls bekannt, dass die Formulierung, die in ihr enthaltenen Fettstoffe, ihre Emulgatoren,

Tenside und ihre Viskosität wesentlichen Einfluss auf die Färbeleistung nehmen. Bei der Modifikation der Formulierung muß eine Verschlechterung der Färbeleistung daher auf jeden Fall vermieden werden.

Beispielsweise schlägt die JP 2007191459 zur Reduktion des Ammoniak-Geruchs in Haarfärbemitteln den Einsatz von kationischen Tensiden, Phophateestern und aliphatischen Alkoholen vor.

WO 2010/015441 A1 beschreibt kosmetische Mittel enthaltend mindestens einen nichtionischen Emulgator mit einem HLB-Wert von kleiner oder gleich 8,0 in Kombination mit bestimmten blauen und roten direktziehenden Farbstoffen. Diese Mittel können als Additive zur Mattierung von Blondiermitteln eingesetzt werden.

In WO 2010/066720 A2 werden Mittel zur Aufhellung von keratinischen Fasern offenbart, welche als Bleichaktivator mindestens ein kationisches Acylpyridinium-Derivat sowie mindestens ein assoziatives Polymer und Wasserstoffperoxid enthalten.

WO 2009/043613 A1 adressiert Mittel zum Aufhellen keratinischer Fasern, enthaltend neben Wasserstoffperoxid mindestens ein kationisches 3,4-Dihydroisochinoliniumderivat.

In WO 2012/079953 A2 schließlich werden Mittel zum Aufhellen keratinischer Fasern beschrieben, in welchen mindestens ein nichtionisches Tensid, mindestens ein anionisches Carbonsäure-Tensid, Wasserstoffperoxid und mindestens ein kationisches Acylpyridiniumderivat zum Einsatz kommen.

Die JP 2003040750 offenbart, dass der Ammoniak-Geruch in Blondiermittel dann besonders gering ist, wenn den Mitteln mindestens 5 % einer kristallinen Komponente zugesetzt wird.

Obwohl die Literatur mehrere Methoden zur Reduktion der durch Ammoniak hervorgerufenen Geruchsbelastungen vorschlägt, so ist dennoch keine Möglichkeit bekannt, denn Ammoniak-Geruch komplett zu unterbinden.

Die Aufgabe der vorliegenden Erfindung war daher die Bereitstellung von nahezu geruchslosen Mitteln zum oxidativen Färben und/oder Aufhellen von Haaren. Zur Erfüllung der an diese Mittel gestellten Leistungsanforderungen sollen sie zwar Ammoniak enthalten, der Ammoniakgeruch soll jedoch komplett überdeckt sein. Insbesondere soll die komplette Überdeckung des Ammoniakgeruchs über einen langen Zeitraum andauern, so dass auch nach einer Lagerung der Mittel (im geschlossenen Gefäß) über mehrere Wochen, im Optimalfall über mehrere Monate, kein Ammoniak-Geruch wahrnehmbar ist. Gleichzeitig sollen die Mittel keine Einbußen hinsichtlich ihrer färberischen Leistung, insbesondere bei ihrer Grauabdeckung und ihrer Waschechtheit, aufweisen. Darüber hinaus soll die Anwendung der Mittel nicht mit einer höheren Haarschädigung verbunden sein.

Weiterhin war es die Aufgabe der vorliegenden Erfindung, den Ammoniakgeruch während der gesamten Anwendungsdauer erfolgreich zu überdecken. Auch nach maximal zweistündigem Kontakt sollte kein Ammoniakgeruch - und darüber hinaus auch kein anderer chemischer Geruch - wahrnehmbar sein.

Überraschenderweise hat sich im Zuge der zu dieser Erfindung führenden Arbeiten herausgestellt, dass es möglich ist, den in den Mitteln zum Färben und/oder Aufhellen enthaltenen Ammoniak geruchlich vollständig zu überdecken bzw. seine geruchliche Wahrnehmung komplett zu verhindern, wenn eine spezielle Kombination aus mehreren verschiedenen Alkalisierungsmitteln eingesetzt wird, und wenn diese verschiedenen Alkalisierungsmittel gleichzeitig mit einem Gemisch aus speziell ethoxylierten Fettalkoholen und höheren Fettalkoholen kombiniert werden.

Ein erster Gegenstand der vorliegenden Erfindung ist daher ein Mittel zum Färben und/oder Aufhellen von keratinischen Fasern, insbesondere menschlichen Haaren, enthaltend in einem kosmetischen Träger

(a) Ammoniak,
(b) ein oder mehrere Alkanolamine aus der Gruppe Monoethanolamin, 2-Amino-2-methylpropanol und Triethanolamin,
(c) ein oder mehrere Alkalisierungsmittel aus der Gruppe Kaliumhydroxid und Natriumhydroxid, der Natriumsilikate und der Kaliumsilikate,
(d) einen oder mehrere ethoxylierte Fettalkohole mit einem Ethoxylierungsgrad von 80 bis 120 und
(e) einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol).

Unter keratinhaltigen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Unter dem erfindungsgemäß verwendeten Begriff "Mittel zum Färben und/oder Aufhellen" von Keratinfasern werden Mitteln zum oxidativen Färben von Haaren und Mittel zum oxidativen Aufhellen bzw. Bleichen von Haaren verstanden.

Oxidative Färbemittel enthalten für die Ausbildung der Färbung Oxidationsfarbstoffvorprodukte, sogenannte Entwickler und Kupplerkomponenten. Entwickler und Kuppler diffundieren getrennt in die Keratinfaser hinein und bilden unter dem Einfluss von Ammoniak als Alkalisierungsmittel und einem Oxidationsmittel (meist Wasserstoffperoxid) in chemischer Reaktion miteinander die eigentlichen Farbstoffe aus. Abhängig von der Menge des eingesetzten Oxidationsmittels wird die Keratinfaser während der Färbung gleichzeitig mehr oder weniger stark aufgehellt, da das Oxidationsmittel nicht nur den Farbstoffbildungsprozess von Entwicklern und Kupplern initiiert, sondern auch die haareigenen Pigmente (Melanine) oxidativ zerstört. Je nach Einsatzmengen der Oxidationsfarbstoffvorprodukte und des Oxidationsmittel kann es sich bei der oxidativen Färbung daher vornehmlich um eine Färbung (mit hohem Farbstoffanteil) oder vornehmlich um eine Aufhellung (mit hohem Anteil an Oxidationsmittel) handeln. In letzterem Fall werden die Oxidationsfarbstoffvorprodukte hauptsächlich zur Nuancierung des Aufhellergebnisses eingesetzt.

Mittel zum oxidativen Aufhellen bzw. Bleichen von Haaren enthalten zur Erzielung eines moderaten Bleicheffektes oft Wasserstoffperoxid als alleiniges Oxidationsmittel, können jedoch - wenn der Wunsch nach einer stärkeren Blondierleistung besteht - auch Oxidationsmittelgemische enthalten. In letzterem Fall wird Wasserstoffperoxid meist in Kombination mit Persulfaten wie Kalium-, Natrium- und/oder Ammoniumpersulfat eingesetzt. Mittel zum oxidativen Aufhellen oder Bleichen können zusätzlich ebenfalls Oxidationsfarbstoffvorprodukte enthalten, der Fokus dieser Mittel liegt jedoch auf der Aufhellen.

In einer bevorzugten Ausführungsform handelt es sich bei den erfindungsgemäßen Mitteln um Mittel zum oxidativen Färben von Haaren.

Die erfindungsgemäßen Mittel enthalten die erfindungswesentlichen Bestandteile in einem kosmetischen Träger, bevorzugt in einem geeigneten wässrigen, alkoholischen oder wässrigalkoholischen Träger. Zum Zwecke der Haarfärbung sind solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind.

Als ersten wesentlichen Formulierungsbestandteil (a) enthalten die erfindungsgemäßen Mittel zum Färben und/oder Aufhellen von Keratinfasern Ammoniak.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

Damit die erfindungsgemäßen Mittel das an sie gestellte Anforderungsprofil im Hinblick auf ihre Farbintensität und ihre Echtheitseigenschaften erfüllen, kann auf den Einsatz von Ammoniak nicht verzichtet werden. Es hat sich jedoch herausgestellt, dass Ammoniak geruchlich komplett überdeckt werden kann, wenn dieser in bestimmten Mengenbereichen in den erfindungsgemäßen Mitteln enthalten ist. Eine vollständige Überdeckung durch die ebenfalls im erfindungsgemäßen Mittel enthaltenen weiteren erfindungswesentlichen Inhaltsstoffe (d) und (e) ist dann möglich, wenn die anwendungsbereiten Mittel Ammoniak (a) in einer Menge von 0,20 bis 1,5 Gew.-%, bevorzugt von 0,25 bis 1,2 Gew.-%, weiter bevorzugt von 0,30 bis 1,0 Gew.-% und besonders bevorzugt von 0,31 bis 0,8 Gew.-% - bezogen auf das Gesamtgewicht des anwendungsbereiten Mittels - enthält.

In einer besonders bevorzugten Ausführungsform ist ein Mittel zum Färben und/oder Aufhellen von keratinischen Fasern daher dadurch gekennzeichnet, dass es Ammoniak (a) in einer Menge von 0,20 bis 1,5 Gew.-%, bevorzugt von 0,25 bis 1,2 Gew.-%, weiter bevorzugt von 0,30 bis 1,0 Gew.-% und besonders bevorzugt von 0,31 bis 0,8 Gew.-% - bezogen auf das Gesamtgewicht des anwendungsbereiten Mittels - enthält.

Die zuvor genannten bevorzugten und besonders bevorzugten Mengenangaben an Ammoniak (a) gehen von reinem Ammoniak als Berechnungsgrundlage aus. Wenn also ganz besonders bevorzugt 0,31 bis 0,8 Gew.-% Ammoniak (a) im anwendungsbereiten Mittel eingesetzt werden, so entspricht dies dem Einsatz einer Menge von 1,24 g bis 3,2 g einer 25 Gew.-%igen Ammoniaklösung im anwendungsbereiten Färbemittel.

Wird Ammoniak in den zuvor beschriebenen Mengenbereichen weiterhin mit den Alkalisierungsmitteln (b) und (c) kombiniert, so können mit den entsprechenden erfindungsgemäßen Mitteln Färbungen mit hoher Farbintensität und ausgezeichneten Echtheitseigenschaften erzeugt werden. Überraschenderweise werden bei dem Einsatz einer Kombination der erfindungswesentlichen Bestandteile (a) bis (e) nicht nur Einbußen der Färbintensität vermieden, sondern die mit diesen Mitteln erzeugten Färbungen weisen darüber hinaus sogar eine verbesserte Grauabdeckung und eine verbesserte Waschechtheit auf.

Als zweiten erfindungswesentlichen Bestandteil (b) enthalten die erfindungsgemäßen Mittel zum Färben und/oder Aufhellen von keratinischen Fasern ein oder mehrere Alkanolamine aus der Gruppe Monoethanolamin (2-Aminoethanol, Formel A), 2-Amino-2-methylpropanol (2-Amino-2-methylpropan-1-ol, Formel B) und Triethanolamin (Formel C).

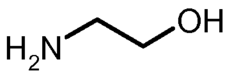

Formel A

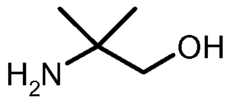

Formel B

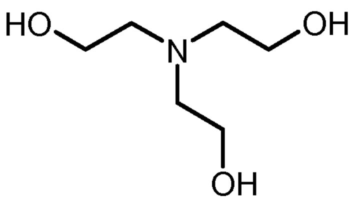

FormelC

Zur Erzielung einer maximalen Geruchsabdeckung und zur Optimierung der Echtheitseigenschaften werden auch das bzw. die Alkanolamine bevorzugt in speziellen Mengenbereichen in eingesetzt. Eine vollständige Abdeckung des Ammoniakgeruchs und gleichzeitig besonders gute Waschechtheiten werden dann erzielt, wenn die Alkanolamine (b) in einer Gesamtmenge von 0,2 bis 6,5 Gew.-%, bevorzugt von 0,5 bis 4,0 Gew.-%, weiter bevorzugt von 0,7 bis 2,5 Gew.-% und besonders bevorzugt von 0,8 bis 1,6 Gew.-% - bezogen auf das Gesamtgewicht des anwendungsbereiten Mittels - enthält.

In einer weiteren bevorzugten Ausführungsform ist ein Mittel zum Färben und/oder Aufhellen von keratinischen Fasern deshalb dadurch gekennzeichnet, dass es ein oder mehrere Alkanolamine (b) aus der Gruppe Monoethanolamin, 2-Amino-2-methylpropanol und Triethanolamin in einer Gesamtmenge von 0,2 bis 6,5 Gew.-%, bevorzugt von 0,5 bis 4,0 Gew.-%, weiter bevorzugt von 0,7 bis 2,5 Gew.-% und besonders bevorzugt von 0,8 bis 1,6 Gew.-% - bezogen auf das Gesamtgewicht des anwendungsbereiten Mittels - enthält.

Für die Erfüllung der erfindungsgemäßen Aufgabenstellung enthalten die erfindungsgemäßen Mittel weiterhin einen oder mehrere Vertreter einer dritten Klasse von Alkalisierungsmitteln (c), die aus der Gruppe Kaliumhydroxid (KOH), Natriumhydroxid (NaOH), der Natriumsilicate und Kaliumsilicate ausgewählt sind.

Kaliumhydroxid und Natriumhydroxid können in Form ihrer Feststoffe eingesetzt werden. Es ist jedoch bevorzugt, wenn diese Hydroxide in Form von wässrigen Lösungen eingesetzt werden. Bei diesen wässrigen Lösungen kann es sich um 50 Gew.-%ige, 40 Gew.-%ige, 30 Gew.-%ige, 20 Gew.-%ige und 10 %ige Lösungen handeln, auch der Einsatz von noch weiter verdünnten wässrigen Lösung in den erfindungsgemäßen Mitteln ist möglich.

Bei Natriumsilikaten im Sinne der vorliegenden Erfindung handelt es sich um chemische Verbindungen, die sich aus Natriumoxid und Siliciumdioxid zusammengesetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel $Na_4SiO_4$, das auch als Natriumorthosilikat bezeichnet wird.

Weitere Beispiele für geeignete Natriumsilikate sind das Dinatriummetasilikat bzw. Natriummetasilikat mit der Summenformel $Na_2SiO_3$, das Dinatriumdisilikat mit der Summenformel $Na_2Si_2O_5$ oder das Dinatriumtrisilikat mit der Summenformel $Na_2Si_3O_7$.

Kaliumsilikate sind entsprechende chemische Verbindungen, die sich aus Kaliumoxid und Siliciumdioxid zusammensetzen, geeignete Kaliumsilicate sind beispielsweise Verbindungen mit den Summenformeln $K_4SiO_4$, $K_2SiO_3$, $K_2Si_2O_5$ und $K_2Si_3O_7$.

Silikate in amporpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwas 150 °C und 5 bar Dampfdruck gelöst, um eine Lösung der Natriumsilikat in Wasser zu erhalten, bei diesen entsprechenden Lösungen handelt es sich um Alkaliwassergläser.

Als Alkaliwassergläser werden aus einer Schmelze erstarrte, glasartige (amorphe) Natrium- und Kaliumsilikate oder ihre wässrigen Lösungen bezeichnet. Je nachdem, ob überwiegend Natriumsilicate oder überwiegend Kaliumsilicate enthalten sind, spricht man von Natronwasserglas oder Kaliwasserglas. Auch Natronwassergläser und Kaliwassergläser sind innerhalb dieser Erfindung von der Definition der Natriumsilikate und der Kaliumsilikate umfasst.

Die molare Zusammensetzung bei Wassergläsern beträgt üblicherweise 2 bis 4 mol SiO2 auf 1 mol Alkalioxid ($Na_2O$ oder $K_2O$).

Ein Beispiel für ein bevorzugtes Natriumsilikat ist Natronwasserglas, welches in Form seiner wässrigen Lösung vorliegt, einen $Na_2O$-Gehalt von 7,5 bis 8,8 Gew.-% und einen SiO2 Gehalt von 25,0 bis 28,5 Gew.-% besitzt und welches die CAS-Nr. 1344-09-5 (Chemical Abstracts Number) besitzt.

Zur Lösung der erfindungsgemäßen Aufgabenstellung hat es sich jedoch als ganz besonders vorteilhaft erwiesen, wenn aus der Gruppe, die aus Kaliumhydroxid (KOH), Natriumhydroxid (NaOH), den Natriumsilicaten und den Kaliumsilicaten gebildet wird, als Alkalisierungmittel (c) Kaliumhydroxid und/oder Natriumhydroxid ausgewählt werden.

In einer weiteren besonders bevorzugten Ausführungsform ist ein Mittel zum Färben und/oder Aufhellen von keratinischen Fasern deshalb dadurch gekennzeichnet, dass es als Alkalisierungsmittel (c) Kaliumhydroxid und/oder Natriumhydroxid enthält.

Von den beiden Alkalisierungsmitteln (c) Kaliumhydroxid und Natriumyhdroxid ist nochmals Kaliumhydroxid

ganz besonders bevorzugt.

In einer weiteren explizit ganz besonders bevorzugten Ausführungsform ist ein Mittel zum Färben und/oder Aufhellen von keratinischen Fasern deshalb dadurch gekennzeichnet, dass es als Alkalisierungsmittel (c) Kaliumhydroxid enthält.'

Die erfindungsgemäßen Mittel sind insbesondere dann besonders geeignet, wenn auch die Vertreter der dritten Klasse von Alkalisierungsmitteln (c), die aus der Gruppe Kaliumhydroxid (KOH), Natriumhydroxid (NaOH), der Natriumsilicate und Kaliumsilicate ausgewählt sind, in speziellen, auf die anderen beiden Alkalisierungsmittelklassen (a) und (b) abgestimmten Mengenbereichen eingesetzt werden.

In einer weiteren ganz besonders bevorzugten Ausführungsform ist ein Mittel zum Färben und/oder Aufhellen von keratinischen Fasern deshalb dadurch gekennzeichnet, dass es ein oder mehrere Alkalisierungsmittel (c) aus der Gruppe Kaliumhydroxid, Natriumhydroxid, der Natriumsilicate und der Kaliumsilikate in einer Gesamtmenge von 0,1 bis 2,5 Gew.-%, bevorzugt von 0,15 bis 1,5 Gew.-%, weiter bevorzugt von 0,2 bis 1,0 Gew.-% und besonders bevorzugt von 0,25 bis 0,5 Gew.-% - bezogen auf das Gesamtgewicht des anwendungsbereiten Mittels - enthält.

Ein bevorzugtes erfindungsgemäßes Mittel enthält in einem kosmetischen Träger

(a) 0,20 bis 1,5 Gew.-%Ammoniak,
(b) 0,2 bis 6,5 Gew.-% Monoethanolamin,
(c) 0,1 bis 2,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,20 bis 1,5 Gew.-% Ammoniak,
(b) 0,2 bis 6,5 Gew.-% Monoethanolamin,
(c) 0,1 bis 2,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,20 bis 1,5 Gew.-% Ammoniak,
(b) 0,2 bis 6,5 Gew.-% 2-Amino-2-methylpropanol,
(c) 0,1 bis 2,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,20 bis 1,5 Gew.-% Ammoniak,
(b) 0,2 bis 6,5 Gew.-% 2-Amino-2-methylpropanol,
(c) 0,1 bis 2,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,25 bis 1,2 Gew.-% Gew.-% Ammoniak,
(b) 0,5 bis 4,0 Gew.-% Monoethanolamin,
(c) 0,15 bis 1,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,25 bis 1,2 Gew.-% Ammoniak,
(b) 0,5 bis 4,0 Gew.-% Monoethanolamin,
(c) 0,15 bis 1,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,25 bis 1,2 Gew.-% Ammoniak,
(b) 0,5 bis 4,0 Gew.-% 2-Amino-2-methylpropanol,
(c) 0,15 bis 1,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,25 bis 1,2 Gew.-% Ammoniak,
(b) 0,5 bis 4,0 Gew.-% 2-Amino-2-methylpropanol,
(c) 0,15 bis 1,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,30 bis 1,0 Gew.-% Ammoniak,
(b) 0,7 bis 2,5 Gew.-% Monoethanolamin,
(c) 0,2 bis 1,0 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,30 bis 1,0 Gew.-% Ammoniak,
(b) 0,7 bis 2,5 Gew.-% Monoethanolamin,
(c) 0,2 bis 1,0 Gew.-% Natriumhydroxid,

(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,30 bis 1,0 Gew.-% Ammoniak,
(b) 0,7 bis 2,5 Gew.-% 2-Amino-2-methylpropanol,
(c) 0,2 bis 1,0 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,30 bis 1,0 Gew.-% Ammoniak,
(b) 0,7 bis 2,5 Gew.-% 2-Amino-2-methylpropanol,
(c) 0,2 bis 1,0 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,35 bis 0,8 Gew.-% Ammoniak,
(b) 0,8 bis 1,6 Gew.-% Monoethanolamin,
(c) 0,25 bis 0,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,35 bis 0,8 Gew.-% Ammoniak,
(b) 0,8 bis 1,6 Gew.-% Monoethanolamin,
(c) 0,25 bis 0,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,35 bis 0,8 Gew.-% Ammoniak,
(b) 0,8 bis 1,6 Gew.-% 2-Amino-2-methylpropanol,
(c) 0,25 bis 0,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Ein weiteres bevorzugtes erfindungsgemäßes Mittel enthält in einem kosmetischen Träger

(a) 0,35 bis 0,8 Gew.-% Ammoniak,
(b) 0,8 bis 1,6 Gew.-% 2-Amino-2-methylpropanol,
(c) 0,25 bis 0,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), wobei alle Mengenangaben auf das Gesamtgewicht des anwendungsbereiten Mittel bezogen sind.

Als vierten erfindungswesentlichen Formulierungsbestandteil (d) enthalten die erfindungsgemäßen Mittel einen oder mehrere ethoxylierte Fettalkohole mit einem Ethoxylierungsgrad von 80 bis 120.

Unter Fettalkoholen sind erfindungsgemäß gesättigte oder ungesättigte, unverzweigte oder verzweigte $C_8$-$C_{28}$-Alkylgruppen mit Hydroxysubstitution zu verstehen. Ungesättigte Fettalkohole können einfach oder mehrfach ungesättigt sein. Bei einem ungesättigten Fettalkohol kann bzw. können deren C-C-Doppelbindung(en) die Cis- oder Trans-Konfiguration aufweisen.

Bevorzugte Fettalkohole sind Octan-1-ol (Octylalkohol, Caprylalkohol), Decan-1-ol (Decylalkohol, Caprinalkohol), Dodecan-1-ol (Dodecylalkohol, Laurylalkohol), Tetradecan-1-ol, (Tetradecylalkohol, Myristylalkohol), Hexadecan-1-ol (Hexadecylalkohol, Cetylalkohol, Palmitylalkohol),

Octadecan-1-ol (Octadecylalkohol, Stearylalkohol), (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Eicosan-1-ol (Eicosylalkohol, Arachylalkohol), (9*Z*)-Eicos-9-en-1-ol (Gadoleylalkohol), (5*Z*,8*Z*,11*Z*,14*Z*)-Eicosa-5,8,11,14-tetraen-1-ol (Arachidonalkohol), Docosan-1-ol (Docosylalkohol, Behenylalkohol), (13*E*)-Docosen-1-ol (Brassidylalkohol) und (13*Z*)-Docos-13-en-1-ol (Erucylalkohol). Innerhalb dieser Gruppe wiederum sind Hexadecan-1-ol (Hexadecylalkohol, Cetylalkohol, Palmitylalkohol) und Octadecan-1-ol (Octadecylalkohol, Stearylalkohol) ganz besonders bevorzugt Fettalkohole.

Zur Ausbildung des erfindungswesentlichen Bestandteils (d) sind diese Fettalkohole mit einem Ethoxylierungsgrad von 80 bis 120 ethoxliert.

Unter Ethoxylierung (auch Oxyethylierung) wird die Reaktion der Fettalkohole mit Ethylenoxid (EO) verstanden. Durch Insertion von 80 bis 120 Gruppierungen des Typs -CH2-CH2-O- pro Fettalkohol Molekül entstehen lineare Polyether, die an einem Kettenende eine Hydroxygruppe und am anderen Kettenende die $C_8$-$C_{28}$-Alkylgruppe des Fettalkohols tragen.

Bevorzugte ethoxylierte Fettalkohole (d) besitzen einen Ethoxylierungsgrad von 90 bis 110. Ganz besonders bevorzugt ist es, wenn ethoxylierte Fettalkohole (d) eingesetzt werden, die einen Ethoxylierungsgrad von 100 besitzen.

In einer weiteren ganz besonders bevorzugten Ausführungsform ist ein Mittel zum Färben und/oder Aufhellen von keratinischen Fasern dadurch gekennzeichnet, dass es als ethoxylierte(n) Fettalkohol(e) (d) mit einem Ethoxylierungsgrad von 80 bis 120 eine oder mehrere Verbindungen der Formel (I) enthält,

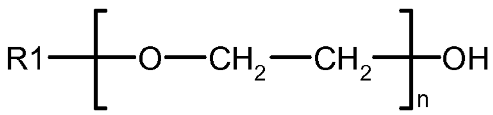

(I)

worin R1 für eine gesättigte oder ungesättigte, unverzweigte oder verzweigte $C_8$-$C_{24}$-Alkylgruppe, bevorzugt für eine gesättigte, unverzeigte $C_{16}$- bis $C_{18}$- Alkylgruppe, steht und
n für eine ganze Zahl von 80 bis 120, bevorzugt für eine ganze Zahl von 90 bis 110 und besonders bevorzugt für die Zahl 100, steht.

Im Zuge der zu dieser Erfindung führenden Arbeiten hat sich gezeigt, dass der Ethoxylierungsgrad des ethoxylierten Fettalkohols (d) überraschenderweise die Fähigkeit des Mittels zur Reduzierung des Ammoniak-Geruchs wesentlich beeinflusst. Aus diesem Grund ist es besonders bevorzugt, wenn als ethoxylierte(r) Fettalkohol(e) (d) ein oder mehrere ethoxylierte Fettalkohole mit einem ganz bestimmten Ethoxylierungsgrad eingesetzt werden.

Ein ganz besonders vorteilhaftes und damit explizit ganz besonders bevorzugtes Mittel zum Färben und/oder Aufhellen von keratinischen Fasern ist dadurch gekennzeichnet, dass es als ethoxylierte(n) Fettalkohol(e) (d) eine oder

mehrere Verbindungen aus der Gruppe

- Hexadecan-1-ol (Hexadecylalkohol, Cetylalkohol, Palmitylalkohol), ethoxyliert mit 90 EO,
- Hexadecan-1-ol (Hexadecylalkohol, Cetylalkohol, Palmitylalkohol), ethoxyliert mit 91 EO,
- Hexadecan-1-ol (Hexadecylalkohol, Cetylalkohol, Palmitylalkohol), ethoxyliert mit 92 EO,
- Hexadecan-1-ol (Hexadecylalkohol, Cetylalkohol, Palmitylalkohol), ethoxyliert mit 93 EO,
- Hexadecan-1-ol (Hexadecylalkohol, Cetylalkohol, Palmitylalkohol), ethoxyliert mit 94 EO,
- Hexadecan-1-ol (Hexadecylalkohol, Cetylalkohol, Palmitylalkohol), ethoxyliert mit 95 EO,
- Hexadecan-1-ol (Hexadecylalkohol, Cetylalkohol, Palmitylalkohol), ethoxyliert mit 96 EO,
- Hexadecan-1-ol (Hexadecylalkohol, Cetylalkohol, Palmitylalkohol), ethoxyliert mit 97 EO,
- Hexadecan-1-ol (Hexadecylalkohol, Cetylalkohol, Palmitylalkohol), ethoxyliert mit 98 EO,
- Hexadecan-1-ol (Hexadecylalkohol, Cetylalkohol, Palmitylalkohol), ethoxyliert mit 99 EO,
- Hexadecan-1-ol (Hexadecylalkohol, Cetylalkohol, Palmitylalkohol), ethoxyliert mit 100 EO,
- Hexadecan-1-ol (Hexadecylalkohol, Cetylalkohol, Palmitylalkohol), ethoxyliert mit 101 EO,
- Hexadecan-1-ol (Hexadecylalkohol, Cetylalkohol, Palmitylalkohol), ethoxyliert mit 102 EO,
- Hexadecan-1-ol (Hexadecylalkohol, Cetylalkohol, Palmitylalkohol), ethoxyliert mit 103 EO,
- Hexadecan-1-ol (Hexadecylalkohol, Cetylalkohol, Palmitylalkohol), ethoxyliert mit 104 EO,
- Hexadecan-1-ol (Hexadecylalkohol, Cetylalkohol, Palmitylalkohol), ethoxyliert mit 105 EO,
- Hexadecan-1-ol (Hexadecylalkohol, Cetylalkohol, Palmitylalkohol), ethoxyliert mit 106 EO,
- Hexadecan-1-ol (Hexadecylalkohol, Cetylalkohol, Palmitylalkohol), ethoxyliert mit 107 EO,
- Hexadecan-1-ol (Hexadecylalkohol, Cetylalkohol, Palmitylalkohol), ethoxyliert mit 108 EO,
- Hexadecan-1-ol (Hexadecylalkohol, Cetylalkohol, Palmitylalkohol), ethoxyliert mit 109 EO,
- Hexadecan-1-ol (Hexadecylalkohol, Cetylalkohol, Palmitylalkohol), ethoxyliert mit 110 EO,
- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

enthält.

Der Ammoniakgeruch der erfindungsgemäßen Mittel zum Färben und/oder Aufhellen von Haaren kann insbesondere dann vollständig während des gesamten Anwendungszeitraums überdeckt werden, wenn die speziell ethoxylierten Fettalkohole (d) in speziellen Mengenbereichen eingesetzt werden.

In einer weiteren bevorzugten Ausführungsform ist ein Mittel zum Färben und/oder Aufhellen von keratinischen Fasern deshalb dadurch gekennzeichnet, dass es einen oder mehrere ethoxylierte Fettalkohole (d) mit einem Ethoxylierungsgrad von 80 bis 120 in einer Gesamtmenge von 0,1 bis 1,5 Gew.-%, bevorzugt von 0,2 bis 1,2 Gew.-%, weiter bevorzugt von 0,3 bis 0,9 Gew.-% und besonders bevorzugt von 0,4 bis 0,8 Gew.-% - bezogen auf das Gesamtgewicht des anwendungsbereiten Mittels - enthält.

Als fünften erfindungswesentlichen Formulierungsbestandteil (e) enthalten die erfindungsgemäßen Mittel schließlich einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol).

In diesem Zusammenhang hat sich herausgestellt, dass die erfindungsgemäßen Mittel insbesondere dann ganz besonders geeignet sind, wenn in ihnen die Fettalkohole (e) Arachylalkohol (Eicosan-1-ol) und/oder Behenylalkohol (Docosan-1-ol) eingesetzt werden.

In kosmetischen Mitteln, welche einen dieser beiden Alkohole - oder ihr Gemisch - enthielten, konnte der Ammoniak über einen besonders langen Zeitraum komplett überdeckt werden. Darüber hinaus ließen sich mit diesen Mittel auch Färbungen mit ausnehmen guten Echtheitseigenschaften, insbesondere eine gute Waschechtheit und eine gute Grauabdeckung, erzielen.

In einer weiteren bevorzugten Ausführungsform ist ein Mittel zum Färben und/oder Aufhellen von keratinischen Fasern dadurch gekennzeichnet, dass es als Fettalkohol (e) Arachylalkohol (Eicosan-1-ol) enthält.

In einer weiteren bevorzugten Ausführungsform ist ein Mittel zum Färben und/oder Aufhellen von keratinischen Fasern dadurch gekennzeichnet, dass es als Fettalkohol (e) Behenylalkohol (Docosan-1-ol) enthält.

In einer weiteren bevorzugten Ausführungsform ist ein Mittel zum Färben und/oder Aufhellen von keratinischen Fasern dadurch gekennzeichnet, dass es als Fettalkohol (e) Arachylalkohol (Eicosan-1-ol) und Behenylalkohol (Docosan-1-ol) enthält.

In einer weiteren ganz besonders bevorzugten Ausführungsform ist ein Mittel zum Färben und/oder Aufhellen von keratinischen Fasern dadurch gekennzeichnet, dass es als Fettalkohole (e) Arachylalkohol (Eicosan-1-ol) und/oder Behenylalkohol (Docosan-1-ol) enthält.

Die Echtheitseigenschaften der mit den erfindungsgemäßen Mitteln erzielbaren Färbungen lassen sich insbesondere dann weiter optimieren, wenn auch die Fettalkohole (e) in speziellen Mengenbereichen eingesetzt werden.

In einer weiteren besonders bevorzugten Ausführungsform ist ein Mittel zum Färben und/oder Aufhellen von keratinischen Fasern daher dadurch gekennzeichnet, dass es den oder die Fettalkohole (e) aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,7 bis 5,0 Gew.-%, bevorzugt von 0,9 bis 4,0 Gew.-%, weiter bevorzugt von 1,1 bis 3,0 Gew.-% und besonders bevorzugt von 1,2 bis 2,8 Gew.-% - bezogen auf das Gesamtgewicht des anwendungsbereiten Mittels - enthält.

In einer weiteren besonders bevorzugten Ausführungsform ist ein erfindungsgemäßes Mittel dadurch gekennzeichnet, dass es in einem kosmetischen Träger

(a) Ammoniak,
(b) Monoethanolamin,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

(e) Behenylalkohol (Docosan-1-ol)enthält.

In einer weiteren besonders bevorzugten Ausführungsform ist ein erfindungsgmäßes Mittel dadurch gekennzeichnet, dass es in einem kosmetischen Träger

(a) Ammoniak,
(b) 2-Amino-2-methylpropanol,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

(e) Behenylalkohol (Docosan-1-ol)enthält.

In einer weiteren besonders bevorzugten Ausführungsform ist ein erfindungsgemäßes Mittel dadurch gekennzeichnet, dass es in einem kosmetischen Träger

(a) Ammoniak,
(b) Monoethanolamin,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,

- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

(e) Arachylalkohol (Eicosan-1-ol) enthält.

In einer weiteren besonders bevorzugten Ausführungsform ist ein erfindungsgmäßes Mittel dadurch gekennzeichnet, dass es in einem kosmetischen Träger

(a) Ammoniak,
(b) 2-Amino-2-methylpropanol,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

(e) Arachylalkohol (Eicosan-1-ol) enthält.

In einer weiteren besonders bevorzugten Ausführungsform ist ein erfindungsgmäßes Mittel dadurch gekennzeichnet, dass es in einem kosmetischen Träger

(a) Ammoniak,
(b) Monoethanolamin,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,

- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

(e) Arachylalkohol (Eicosan-1-ol) und Behenylalkohol (Docosan-1-ol) enthält.

In einer weiteren besonders bevorzugten Ausführungsform ist ein erfindungsgmäßes Mittel dadurch gekennzeichnet, dass es in einem kosmetischen Träger

(a) Ammoniak,
(b) 2-Amino-2-methylpropanol,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO,

(e) Arachylalkohol (Eicosan-1-ol) und Behenylalkohol (Docosan-1-ol) enthält.

Da es sich bei den erfindungsgemäßen Mitteln um Mittel zum oxidativen Färben von Haaren handelt, enthalten diese zur Ausbildung der Farbstoffe daher zusätzlich Oxidationsfarbstoffvorprodukte.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)-phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Di-hydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform enthalten die erfindungsgemäßen Mittel zusätzlich ein oder mehrere Oxidationsfarbstoffvorprodukte in einer Gesamtmenge von 0,01 bis 4,0 Gew.-%, bevorzugt von 0,1 bis 3,5 Gew.-%, weiter bevorzugt von 0,6 bis 3,1 Gew.-% und ganz besonders bevorzugt von 1,2 bis 2,2 Gew.-%, bezogen auf das Gesamtgewicht des anwendungsbereiten Mittels.

In einer weiteren bevorzugten Ausführungsform enthalten die erfindungsgemäßen Mittel zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf die gesamte Anwendungszubereitung, eingesetzt.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitro-phenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Zusätzlich zu den vorgenannten Inhaltsstoffen können die erfindungsgemäßen Mittel weiterhin die für oxidative Färbemittel üblichen Inhaltsstoffe enthalten.

Die Ausbildung der Farbstoffe in oxidativen Färbemitteln erfolgt erst durch den Einfluss eines Oxidationsmittels, üblicherweise wird hierfür Wasserstoffperoxid verwendet. In einer bevorzugten Ausführungsform wird das Wasserstoffperoxid als wässrige Lösung verwendet. Erfindungsgemäß bevorzugte Oxidationsmittelzubereitungen sind dadurch gekennzeichnet, dass sie 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges $H_2O_2$) enthalten.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Weiterhin können die erfindungsgemäßen Mittel zusätzlich Polymere und/oder Verdicker enthalten. Als Polymere können kationische, anionische und/oder zwitterionische Polymere Einsatz finden. Beispiele für geeignete anionische Polymere sind beispielsweise unter dem Warenzeichnen Carbopol® oder Rheothik®11-80 im Handel erhältlich. Auch die Polymere, die unter dem INCI-Namen Acrylates Copolymere vertrieben werden, sind geeignete anionische Polymere. Ein bevorzugtes Handelsprodukt ist beispielsweise Aculyn® 33 der Firma Rohm & Haas. Weitere bevorzugte anionische Polymere werden von der Firma Rohm & Haas unter der Handelsbezeichnung Aculyn® 22 sowie von der Firma National Starch unter den Handelsbezeichnungen Structure® 2001 und Structure® 3001 vertrieben.

Geeignete zusätzlich einsetzbare kationische Polymere sind beispielsweise Polyquaternium-24 (Handelsprodukt z. B. Quatrisoft® LM 200), Copolymer 845 (Hersteller: ISP), Gaffix® VC 713 (Hersteller: ISP), Gafquat®ASCP 1011, Gafquat®HS 110, Luviquat®8155 und Luviquat® MS 370.

Auch natürlich vorkommende Verdickungsmittel können auch nichtionische Guargums, wie beispielsweise sowohl modifizierte (z.B. Jaguar® HP8, Jaguar® HP60, Jaguar® HP120, Jaguar® DC 293 und Jaguar® HP105) als auch unmodifizierte Guargums (z.B. Jaguar® C) zum Einsatz kommen. Weitere geeignete Verdickungsmittel sind die Skleroglucangums oder Xanthangums, Gums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, Cellulosederivate, wie beispielsweise Methylcellulose, Carboxyalkylcellulosen und Hydroxyalkylcellulosen.

Weitere anionische, kationische oder amphotere Tenside können ebenfalls in den erfindungsgemäßen Mitteln enthalten sein. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Besonders bevorzugte amphotere Tenside sind das N-Kokosalkylaminopropionat, das Kokosacylaminoethylaminopropionat und das $C_{12}$-$C_{18}$-Acylsarcosin.

Bevorzugte zusätzlich enthaltene kationische Tenside sind beispielsweise Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.

Ferner können die erfindungsgemäßen Mittel weitere Wirk-, Hilfs- und Zusatzstoffe enthalten, wie beispielsweise nichtionische Polymere, Silikone, kationische Polymere, Strukturanden, Lösungsmittel und Vermittler, faserstrukturverbessernde Wirkstoffe, Entschäumer wie Silikone, Antischuppenwirkstoffe, Proteinhydrolysate, pflanzliche Öle, beispielsweise Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl, Lichtschutzmittel, Substanzen zur Einstellung des pH-Wertes, wie beispielsweise übliche Säuren, insbesondere Genusssäuren, Vitamine, Provitamine und Vitaminvorstufen, Pflanzenextrakte, Konsistenzgeber, Wachse, weitere Quell- und Penetrationsstoffe, Perlglanzmittel wie Ethylenglykolmono- und - distearat sowie PEG-3-distearat, Treibmittel wie Propan-Butan-Gemische, $N_2O$, Dimethylether, $CO_2$ und Luft und Antioxidantien.

Die Auswahl dieser weiteren Stoffe wird der Fachmann gemäß der gewünschten Eigenschaften der Mittel treffen.

Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Färbe- und Aufhellprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 12, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Bei den erfindungsgemäßen Mitteln handelt es sich um Mittel zum oxidativen Färben und/oder Aufhellen von Haaren. Im anwendungsbereiten Mittel reagieren die Oxidationsfarbstoffvorprodukte mit dem Oxidationsmittel unter Ausbildung der eigentlichen Farbstoffe. Üblicherweise werden die erfindungsgemäßen Mittel daher als Mehrkomponentenmittel, meist als Zweikomponenten-Mittel, konfektioniert. Die erste Komponente beinhaltet hierbei die Oxidationsfarbstoffvorprodukte und das Alkalisierungsmittel (Zubereitung A), welche kurz vor der Anwendung mit einer zweiten Komponenente enthaltend das Oxidationsmittel (Zubereitung B) vermischt wird. Üblicherweise werden beide Komponenten im Verhältnis 1:3 bis 3:1 miteinander vermischt. Bei dieser Mischung der Komponente enthaltend Farbcreme/Alkalisierungsmittel (Zubereitung A) und der Komponente enthaltend Oxidationsmittel (Zubereitung B) spricht man von der Anwendungsmischung oder dem anwendungsbereiten Mittel. Alle Mengenangaben mit Bezugnahme auf das "anwendungsbereite Mittel" beziehen sich auf die anwendungsbereite Mischung aus der Komponente enthaltend Farbcreme/Alkalisierungsmittel und der Komponente enthaltend Oxidationsmittel.

Ein weiterer Gegenstand der vorliegenden Erfindung ist daher ein anwendungsbereites Mittel zum Färben und/oder Aufhellen von keratinischen Fasern, welches dadurch gekennzeichnet ist, dass es unmittelbar vor der Anwendung durch Vermischen der Zubereitungen (A) und (B) hergestellt wird, wobei

- es sich bei der Zubereitung (A) um ein Mittel des ersten Erfindungsgegenstandes handelt,
- es sich bei der Zubereitung (B) um ein Mittel handelt, das in einem kosmetischen Träger Wasserstoffperoxid enthält, und
- das durch Vermischen der Zubereitungen (A) und (B) hergestellte anwendungsbereite Mittel Wasserstoffperoxid in einer Menge von 0,5 bis 8,0 Gew.-%, bevorzugt 1,5 bis 6,5 Gew.-%, weiter bevorzugt 2,2 bis 4,5 Gew.-% und besonders bevorzugt 3,0 bis 3,6 Gew.-% (berechnet als 100 %- iges Wasserstoffperoxid) - bezogen auf das Gesamtgewicht des anwendungsbereiten Mittels - enthält.

Ein weiterer Gegenstand der vorliegenden Erfindung ist ein Verfahren zum Färben und/oder Aufhellen von keratinischen Fasern, welches dadurch gekennzeichnet ist, dass

- gewünschtenfalls ein Vorbehandlungsmittel M1 auf die Faser aufgebracht wird, dann
- ein Färbe- und/oder Aufhellmittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von 2 bis 25 Minuten wieder abgespült wird,

wobei das Mittel M2 ein erfindungsgemäßes Mittel ist.

Bezüglich weiterer bevorzugter Ausführungsformen der erfindungsgemäßen Verfahren und Verwendungen gilt mutatis mutandis das zu den erfindungsgemäßen Mitteln Gesagte.

## Beispiele

### 1. Ausfärbungen und Bestimmung der Waschechtheiten

Es wurden die folgenden Rezepturen hergestellt.

| Formulierungsbestandteile (Farbcreme) | V1 (Gew.-%) | E1 (Gew.-%) |
|---|---|---|
| Cetylalkohol | 8,10 | 5,70 |
| Lanette 22 (INCI: Behenylalcohol) | --- | 2,40 |
| Wacker Belsil ADM 1650 (INCI: Amodimethicone) | 0,5 | 0,5 |
| Eumulgin B 1 (INCI: Ceteareth-12) | 1,2 | --- |
| Eumulgin B 3 (INCI: Ceteareth-30) | --- | 1,2 |
| Eumulgin B 2 (INCI: Ceteareth-20) | 0,6 | --- |
| Brij S 100 PA (Stearyl alcohol ethoxylated (100 EO)) | --- | 0,6 |
| Cutina GMS (INCI: Glyceryl Stearate) | 0,6 | 0,6 |
| Genamin STAC (INCI: Steartrimonium Chloride) | 1,75 | 1,75 |
| Propylene Glycol | 6,0 | 6,0 |
| p-Toluylendiamin Sulfat | 1,50 | 1,50 |

(fortgesetzt)

| Formulierungsbestandteile (Farbcreme) | V1 (Gew.-%) | E1 (Gew.-%) |
|---|---|---|
| Resorcin | 0,58 | 0,58 |
| m-Aminophenol | 0,16 | 0,16 |
| 3-Amino-2-methylamino-6-methoxypyridin | 0,05 | 0,05 |
| Kaliumhydroxid (50 %ig) | 0,7 | 0,7 |
| Ethylendiamintetraessigsäure, Tetranatriumsalz | 0,20 | 0,20 |
| Natriumsulfit (wasserfrei) | 0,30 | 0,30 |
| Vitamin C | 0,05 | 0,05 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer) 20 Gew.-%ige wässrige Lösung | 2,00 | 2,00 |
| Monoethanolamin | 2,00 | 2,00 |
| Ammoniak (25 Gew.-%ige wässrige Lösung) | 5,80 | 5,80 |
| Parfum | 0,40 | 0,40 |
| Wasser | ad 100 | ad 100 |

Bei V1 handelt es sich um eine Vergleichsrezeptur, E1 ist eine erfindungsgemäße Formulierung. Die Farbcremes wurden jeweils im Verhältnis 1:1 mit der folgenden Oxidationsmittelformierung (OX1) vermischt.

| Formulierungsbestandteile | OX1 (Gew.-%) |
|---|---|
| Phosphorsäure 85 %ig | 0,04 |
| Wasserstoffperoxid (50 %ige, wässrige Lösung) | 12,00 |
| Emulgade F (INCI: Cetearylalcohol, PEG-40 Castor Oil, Sodium Cetearyl sulfate) | 2,10 |
| Natriumbenzoat | 0,04 |
| Dinatriumpyrophosphat | 0,30 |
| Ethylendiamintetraacetat, Dinatriumsalz | 0,15 |
| Wasser | ad 100 |

Die auf diese Weise hergestellten Anwendungmischungen wurden mit einer Aplicette auf Haarsträhnen (Büffelbauchhaar) aufgetragen und dort für einen Zeitraum von 10 bis 30 Minuten belassen. Anschließend wurde die Anwendungsmischung mit einem Shampoo ausgespült und getrocknet. Es wurden dunkelbraune Färbungen erhalten. Dann wurden die Haarsträhnen farbmetrisch vermessen (Messung der Lab-wert) Danach wurden die Haarsträhnen 6 mal, 12 mal und 18 mal gewaschen und jeweils nach 6, 12, und 18 Haarwäschen (HW) erneut farbmetrisch vermessen. Aus den Lab-Werten wurde jeweils der ΔE-Wert (Farbabstand) nach folgender Formel berechnet:

$$\Delta E = \sqrt{(L_0 - L_x)^2 + (a_0 - a_x)^2 + (b_0 - b_x)^2}$$

$L_0$, $a_0$, $b_0$ Farbmetrikwerte nach 0 Haarwäschen
$L_x$, $a_x$, $b_x$ Farbmetrikwerte jeweils nach 6, 12 bwz. 18 Haarwäschen

Tabelle 1: Waschechtheiten, Anwendungsdauer 10 min

| | ΔE | Haarwäschen (HW) |
|---|---|---|
| V1 + OX1 | --- | 0 |
| E1 + OX1 | --- | 0 |
| V1 + OX1 | 1,80 | 6 |
| E1 + OX1 | 0,96 | 6 |
| V1 + OX1 | 2,53 | 12 |
| E1 + OX1 | 2,22 | 12 |
| V1 + OX1 | 3,11 | 18 |
| E1 + OX1 | 2,74 | 18 |

Tabelle 2: Waschechtheiten, Anwendungsdauer 20 min

| | ΔE | Haarwäschen (HW) |
|---|---|---|
| V1 + OX1 | --- | 0 |
| E1 + OX1 | --- | 0 |
| V1 + OX1 | 1,77 | 6 |
| E1 + OX1 | 1,13 | 6 |
| V1 + OX1 | 2,00 | 12 |
| E1 + OX1 | 1,64 | 12 |
| V1 + OX1 | 1,78 | 18 |
| E1 + OX1 | 1,08 | 18 |

Tabelle 3: Waschechtheiten, Anwendungsdauer 30 min

| | ΔE | Haarwäschen (HW) |
|---|---|---|
| V1 + OX1 | --- | 0 |
| E1 + OX1 | --- | 0 |
| V1 + OX1 | 1,13 | 6 |
| E1 + OX1 | 0,60 | 6 |
| V1 + OX1 | 7,40 | 12 |
| E1 + OX1 | 4,98 | 12 |
| V1 + OX1 | 1,48 | 18 |
| E1 + OX1 | 0,92 | 18 |

Je größer der ΔE-Wert ist, desto größer ist der Farbabstand (Farbunterschied) der jeweiligen Strähne bei Vergleich ihrer Farbe vor und nach den Haarwäschen. Je größer der ΔE-Wert ist, desto schlechter ist damit die Waschechtheit der entsprechenden Färbung.

Es zeigte sich, dass die Waschechtheiten der mit den erfindungsgemäßen Formulierungen erhaltenen Färbungen unabhängig von der Anwendungsdauer (10 min, 20 min und 30 min) durchweg besser waren als die mit den Vergleichsformulierungen erhaltenen Färbungen.

2. Bestimmung des Ammoniakgeruchs während der Anwendung

Die zuvor hergestellten Anwendungsmischungen (V1 + OX1, E1 + OX1) wurden jeweils auf den Kopf eines Probanden aufgetragen. Während des Anwendungszeitraums wurde der Ammoniak-Geruch von jeweils 5 geschulten Personen zu verschiedenen Zeitpunkten (direkt nach der Anwendung nach 0 min, nach 10 min, nach 20 min und nach 30 min.) bewertet. Die Bewertung erfolgte blind, was bedeutet, dass den Personen, die die Bewertung vornahmen, nicht bekannt war, welche Rezeptur sie gerade bewerteten. Aus den Einzelbewertungen wurde jeweils der Mittelwert gebildet.
Der Ammoniak-Geruch wurde auf einer Skala von 0 (quasi kein Geruch wahrnehmbar) bis 10 (sehr starker Ammoniak-Geruch) bewertet.

Tabelle 4: Ammoniak-Geruch während der Anwendung (Anwendungsmischung)

| | nach 0 min. | nach 10 min. | nach 20 min. | nach 30 min. |
|---|---|---|---|---|
| V1 + OX1 | 5 | 3,5 | 3,0 | 1,5 |
| E1 + OX1 | 0 - 1 | 0 - 1 | 0 - 1 | 0 - 1 |

Es zeigt sich, dass der Ammoniak-Geruch bei Anwendung der erfindungsgemäßen Formulierung sowohl direkt nach dem Auftrag der Formulierungen als auch nach einem Zeitraum von 10 Minuten, 20 Minuten und 30 Minuten als deutlich reduziert wahrgenommen wurde.

3. Bestimmung des Ammoniakgeruchs während der Lagerung

Die Formulierungen V1 und E1 wurden in Tuben abgefüllt und für mehrere Wochen bei 40 °C im einem Lagerschrank gelagert. Jeweils direkt bei Lagerbeginn und nach 2 Wochen, nach 4 Wochen, nach 8 Wochen und nach 12 Wochen wurde der Geruch der Tuben nach der oben beschriebenen Methode bewertet.
Der Ammoniak-Geruch wurde auf einer Skala von 0 (quasi kein Geruch wahrnehmbar) bis 10 (sehr starker Ammoniak-Geruch) bewertet.

Tabelle 5: Ammoniak-Geruch der Farbcremes während der Lagerung bei + 40 °C

| Farbcremes | V1 | E1 |
|---|---|---|
| Lagerbeginn | etwas Ammoniak-Geruch wahrnehmbar (Note 4) | quasi kein Ammoniak-Geruch (Note 0 - 1) |
| Lagerung 2 Wochen (+ 40 °C) | Ammoniak-Geruch deutlich wahrnehmbar (Note 5) | quasi kein Ammoniak-Geruch (Note 0 - 1) |
| Lagerung 4 Wochen (+ 40 °C) | wenig Ammoniak-Geruch (Note 2-3) | quasi kein Ammoniak-Geruch (Note 0 - 1) |
| Lagerung 8 Wochen (+ 40 °C) | minimaler Ammoniak-Geruch (Note 1) | quasi kein Ammoniak-Geruch (Note 0 - 1) |
| Lagerung 12 Wochen (+ 40 °C) | minimaler AmmoniakGeruch (Note 1) | quasi kein Ammoniak-Geruch (Note 0 - 1) |

Der Ammoniak-Geruch wurde auf einer Skala von 0 (quasi kein Geruch wahrnehmbar) bis 10 (sehr starker Ammoniak-Geruch) bewertet.

3. Beispielformulierungen

| Formulierungsbestandteile (Farbcreme) | 1 (Gew.-%) | 2 (Gew.-%) |
|---|---|---|
| Cetylalkohol | 3,6 | 4,6 |
| Lanette 22 (INCI: Behenylalcohol) | 2,4 | 3,0 |

(fortgesetzt)

| Formulierungsbestandteile (Farbcreme) | 1 (Gew.-%) | 2 (Gew.-%) |
|---|---|---|
| Paraffinum Liquidum | 2,1 | 2,1 |
| Eumulgin B 3 (INCI: Ceteareth-30) | 1,2 | 1,8 |
| Brij S 100 PA (Stearyl alcohol ethoxylated (100 EO)) | 0,6 | 1,0 |
| Cutina GMS (INCI: Glyceryl Stearate) | 0,6 | 0,6 |
| Propylenglycol | 6,0 | 6,0 |
| p-Toluylendiamin Sulfat | 1,50 | 1,50 |
| Resorcin | 0,58 | 0,58 |
| m-Aminophenol | 0,16 | 0,16 |
| 3-Amino-2-methylamino-6-methoxypyridin | 0,05 | 0,05 |
| Kaliumhydroxid (50 %ig) | 0,7 | 0,7 |
| Ethylendiamintetraessigsäure, Tetranatriumsalz | 0,20 | 0,20 |
| Natriumsulfit (wasserfrei) | 0,30 | 0,30 |
| Vitamin C | 0,05 | 0,05 |
| Produkt W 37194 (N,N,N-Trimethyl-3-[(1-oxo-2-propenyl)amino]-1-propanaminiumchloride, polymer with sodium 2-propenoate) (INCI: Acrylamidopropyltrimonium chloride /Acrylate Copolymer) 20 Gew.-%ige wässrige Lösung | 3,75 | 3,0 |
| Monoethanolamin | 2,00 | 2,00 |
| Ammoniak (25 Gew.-%ige wässrige Lösung) | 5,80 | 5,80 |
| Parfum | 0,40 | 0,40 |
| Wasser | ad 100 | ad 100 |

Die Farbcremes 1 und 2 wurden jeweils im Verhälnis 1:1 mit der Oxidationsmittelformierung (OX 2) vermischt und auf Haaren ausgefärbt.

| Formulierungsbestandteile | OX2 (Gew.-%) |
|---|---|
| Natriumbenzoat | 0,04 |
| Dipicolinsäure (Pyridin-2,6-dicarbonsäure) | 0,10 |
| Dinatriumpyrophosphat | 0,10 |
| Kaliumhydroxid (50 %ig) | 0,19 |
| Propylenglycol | 0,50 |
| Paraffinum Liquidum | 2,00 |
| Cetearylalkohol | 3,40 |
| Ceteareth-20 | 1,00 |
| Wasserstoffperoxid (50 %ige wässrige Lösung) | 12,20 |
| Wasser | ad 100 |

Beide Anwendungsmischungen (Farbcreme 1 + OX2, Farbcreme 2 + OX2) zeichneten sich während des gesamten Anwendungszeitraums durch einen reduzierten Ammoniakgeruch aus.

**Patentansprüche**

1. Mittel zum Färben und/oder Aufhellen von keratinischen Fasern, insbesondere menschlichen Haaren, enthaltend in einem kosmetischen Träger

   (a) Ammoniak,
   (b) ein oder mehrere Alkanolamine aus der Gruppe Monoethanolamin, 2-Amino-2-methylpropanol und Triethanolamin,
   (c) ein oder mehrere Alkalisierungsmittel aus der Gruppe Kaliumhydroxid, Natriumhydroxid, der Natriumsilikate und der Kaliumsilikate,
   (d) einen oder mehrere ethoxylierte Fettalkohole mit einem Ethoxylierungsgrad von 80 bis 120,
   (e) einen oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol).

2. Mittel nach Anspruch 1, **dadurch gekennzeichnet, dass** es Ammoniak (a) in einer Menge von 0,20 bis 1,5 Gew.-%, bevorzugt von 0,25 bis 1,2 Gew.-%, weiter bevorzugt von 0,30 bis 1,0 Gew.-% und besonders bevorzugt von 0,31 bis 0,8 Gew.-% - bezogen auf das Gesamtgewicht des anwendungsbereiten Mittels - enthält.

3. Mittel nach einem der Ansprüche 1 oder 2, **dadurch gekennzeichnet, dass** es ein oder mehrere Alkanolamine (b) aus der Gruppe Monoethanolamin, 2-Amino-2-methylpropanol und Triethanolamin in einer Gesamtmenge von 0,2 bis 6,5 Gew.-%, bevorzugt von 0,5 bis 4,0 Gew.-%, weiter bevorzugt von 0,7 bis 2,5 Gew.-% und besonders bevorzugt von 0,8 bis 1,6 Gew.-% - bezogen auf das Gesamtgewicht des anwendungsbereiten Mittels - enthält.

4. Mittel nach einem der Ansprüche 1 bis 3, **dadurch gekennzeichnet, dass** es als Alkalisierungsmittel Natriumhydroxid und/oder Kaliumhydroxid enthält.

5. Mittel nach einem der Ansprüche 1 bis 4, **dadurch gekennzeichnet, dass** es ein oder mehrere Alkalisierungsmittel (c) aus der Gruppe Kaliumhydroxid, Natriumhydroxid, der Natriumsilicate und der Kaliumsilikate in einer Gesamtmenge von 0,1 bis 2,5 Gew.-%, bevorzugt von 0,15 bis 1,5 Gew.-%, weiter bevorzugt von 0,2 bis 1,0 Gew.-% und besonders bevorzugt von 0,25 bis 0,5 Gew.-% - bezogen auf das Gesamtgewicht des anwendungsbereiten Mittels - enthält.

6. Mittel nach einem der Ansprüche 1 bis 5, **dadurch gekennzeichnet, dass** es als ethoxylierte(n) Fettalkohol(e) (d) mit einem Ethoxylierungsgrad von 80 bis 120 eine oder mehrere Verbindungen der Formel (I) enthält,

   $$R1-\left[O-CH_2-CH_2\right]_n-OH \qquad (I)$$

   worin R1 für eine gesättigte oder ungesättigte, unverzweigte oder verzweigte $C_8$-$C_{24}$-Alkylgruppe, bevorzugt für eine gesättigte, unverzweigte $C_{16}$- oder $C_{18}$- Alkylgruppe, steht und
   n für eine ganze Zahl von 80 bis 120, bevorzugt für eine ganze Zahl von 90 bis 110 und besonders bevorzugt für die Zahl 100, steht.

7. Mittel nach einem der Ansprüche 1 bis 6, **dadurch gekennzeichnet, dass** es einen oder mehrere ethoxylierte Fettalkohole (d) mit einem Ethoxylierungsgrad von 80 bis 120 in einer Gesamtmenge von 0,1 bis 1,5 Gew.-%, bevorzugt von 0,2 bis 1,2 Gew.-%, weiter bevorzugt von 0,3 bis 0,9 Gew.-% und besonders bevorzugt von 0,4 bis 0,8 Gew.-% - bezogen auf das Gesamtgewicht des anwendungsbereiten Mittels - enthält.

8. Mittel nach einem der Ansprüche 1 bis 7, **dadurch gekennzeichnet, dass** es als Fettalkohole (e) Arachylalkohol (Eicosan-1-ol) und/oder Behenylalkohol (Docosan-1-ol) enthält.

9. Mittel nach einem der Ansprüche 1 bis 8, **dadurch gekennzeichnet, dass** es den oder die Fettalkohole (e) aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol) in einer Gesamtmenge von 0,7 bis 5,0 Gew.-%, bevorzugt von 0,9 bis 4,0 Gew.-%, weiter bevorzugt von 1,1 bis 3,0 Gew.-% und besonders bevorzugt von 1,2 bis 2,8 Gew.-% - bezogen auf das Gesamtgewicht des anwendungsbereiten Mittels - enthält.

**10.** Mittel nach einem der Ansprüche 1 bis 9, **dadurch gekennzeichnet, dass** es

(a) 0,20 bis 1,5 Gew.-%Ammoniak,
(b) 0,2 bis 6,5 Gew.-% Monoethanolamin,
(c) 0,1 bis 2,5 Gew.-% Kaliumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol), enthält, wobei alle Mengenangaben auf das Gesamtgewicht des anwendungsbereiten Mittels bezogen sind.

**11.** Mittel nach einem der Ansprüche 1 bis 10, **dadurch gekennzeichnet, dass** es

(a) 0,20 bis 1,5 Gew.-% Ammoniak,
(b) 0,2 bis 6,5 Gew.-% Monoethanolamin,
(c) 0,1 bis 2,5 Gew.-% Natriumhydroxid,
(d) einen oder mehrere ethoxylierte Fettalkohole mit einem Ethoxylierungsgrad von 80 bis 120 und
(e) ein oder mehrere Fettalkohole aus der Gruppe Arachylalkohol (Eicosan-1-ol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Behenylalkohol (Docosan-1-ol), Erucylalkohol ((13*Z*)-Docos-13-en-1-ol) und Brassidylalkohol ((13*E*)-Docosen-1-ol) enthält, wobei alle Mengenangaben auf das Gesamtgewicht des anwendungsbereiten Mittels bezogen sind.

**12.** Mittel nach einem der Ansprüche 1 bis 11, **dadurch gekennzeichnet, dass** es

(a) Ammoniak,
(b) Monoethanolamin,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO, und

(e) Behenylalkohol (Docosan-1-ol)enthält.

**13.** Mittel nach einem der Ansprüche 1 bis 12, **dadurch gekennzeichnet, dass** es

(a) Ammoniak,
(b) Monoethanolamin,
(c) Kaliumhydroxid,
(d) einen oder mehrere ethoxylierte Fettalkohole mit einem Ethoxylierungsgrad von 80 bis 120, die ausgewählt sind aus der Gruppe

- Octadecan-1-ol (Octadecylalkohol, Stearylalkohol), ethoxyliert mit 90 EO,
- Octadecan-1-ol (Octadecylalkohol, Stearylalkohol), ethoxyliert mit 91 EO,
- Octadecan-1-ol (Octadecylalkohol, Stearylalkohol), ethoxyliert mit 92 EO,
- Octadecan-1-ol (Octadecylalkohol, Stearylalkohol), ethoxyliert mit 93 EO,
- Octadecan-1-ol (Octadecylalkohol, Stearylalkohol), ethoxyliert mit 94 EO,
- Octadecan-1-ol (Octadecylalkohol, Stearylalkohol), ethoxyliert mit 95 EO,
- Octadecan-1-ol (Octadecylalkohol, Stearylalkohol), ethoxyliert mit 96 EO,
- Octadecan-1-ol (Octadecylalkohol, Stearylalkohol), ethoxyliert mit 97 EO,
- Octadecan-1-ol (Octadecylalkohol, Stearylalkohol), ethoxyliert mit 98 EO,
- Octadecan-1-ol (Octadecylalkohol, Stearylalkohol), ethoxyliert mit 99 EO,
- Octadecan-1-ol (Octadecylalkohol, Stearylalkohol), ethoxyliert mit 100 EO,
- Octadecan-1-ol (Octadecylalkohol, Stearylalkohol), ethoxyliert mit 101 EO,
- Octadecan-1-ol (Octadecylalkohol, Stearylalkohol), ethoxyliert mit 102 EO,
- Octadecan-1-ol (Octadecylalkohol, Stearylalkohol), ethoxyliert mit 103 EO,
- Octadecan-1-ol (Octadecylalkohol, Stearylalkohol), ethoxyliert mit 104 EO,
- Octadecan-1-ol (Octadecylalkohol, Stearylalkohol), ethoxyliert mit 105 EO,
- Octadecan-1-ol (Octadecylalkohol, Stearylalkohol), ethoxyliert mit 106 EO,
- Octadecan-1-ol (Octadecylalkohol, Stearylalkohol), ethoxyliert mit 107 EO,
- Octadecan-1-ol (Octadecylalkohol, Stearylalkohol), ethoxyliert mit 108 EO,
- Octadecan-1-ol (Octadecylalkohol, Stearylalkohol), ethoxyliert mit 109 EO,
- Octadecan-1-ol (Octadecylalkohol, Stearylalkohol), ethoxyliert mit 110 EO, und

(e) Arachylalkohol (Eicosan-1-ol) und Behenylalkohol (Docosan-1-ol) enthält.

**14.** Anwendungsbereites Mittel zum Färben und/oder Aufhellen von keratinischen Fasern, **dadurch gekennzeichnet, dass** es unmittelbar vor der Anwendung durch Vermischen der Zubereitungen (A) und (B) hergestellt wird, wobei

- es sich bei der Zubereitung (A) um ein Mittel der Ansprüche 1 bis 13 handelt,
- es sich bei der Zubereitung (B) um ein Mittel handelt, das in einem kosmetischen Träger Wasserstoffperoxid enthält, und
- das durch Vermischen der Zubereitungen (A) und (B) hergestellte anwendungsbereite Mittel Wasserstoffperoxid in einer Menge von 0,5 bis 8,0 Gew.-%, bevorzugt 1,5 bis 6,5 Gew.-%, weiter bevorzugt 2,2 bis 4,5 Gew.-% und besonders bevorzugt 3,0 bis 3,6 Gew.-% (berechnet als 100 %-iges Wasserstoffperoxid) - bezogen auf das Gesamtgewicht des anwendungsbereiten Mittels - enthält.

## Claims

**1.** An agent for dyeing and/or lightening keratinic fibers, in particular human hair, containing, in a cosmetic carrier:

(a) ammonia,
(b) one or more alkanolamines from the group consisting of monoethanolamine, 2-amino-2-methylpropanol, and triethanolamine,
(c) one or more alkalizing agents from the group consisting of potassium hydroxide, sodium hydroxide, sodium silicates, and potassium silicates,
(d) one or more ethoxylated fatty alcohols having a degree of ethoxylation of from 80 to 120,
(e) one or more fatty alcohols from the group consisting of arachyl alcohol (eicosan-1-ol), gadoleyl alcohol ((9*Z*)-eicos-9-en-1-ol), arachidonyl alcohol ((5*Z*,8*Z*,11*Z*,14*Z*)-eicosa-5,8,11,14-tetraen-1-ol), behenyl alcohol (docosan-1-ol), erucyl alcohol ((13*Z*)-docos-13-en-1-ol), and brassidyl alcohol ((13*E*)-docosen-1-ol).

2. The agent according to claim 1, **characterized in that** it contains ammonia (a) an amount of from 0.20 to 1.5 wt.%, preferably from 0.25 to 1.2 wt.%, more preferably from 0.30 to 1.0 wt.% and particularly preferably from 0.31 to 0.8 wt.%, based on the total weight of the ready-to-use agent.

3. The agent according to either claim 1 or claim 2, **characterized in that** it contains one or more alkanolamines (b) from the group consisting of monoethanolamine, 2-amino-2-methylpropanol and triethanolamine in a total amount of from 0.2 to 6.5 wt.%, preferably from 0.5 to 4.0 wt.%, more preferably from 0.7 to 2.5 wt.% and particularly preferably from 0.8 to 1.6 wt.%, based on the total weight of the ready-to-use agent.

4. The agent according to one of claims 1 to 3, **characterized in that** it contains sodium hydroxide and/or potassium hydroxide as the alkalizing agent.

5. The agent according to one of claims 1 to 4, **characterized in that** it contains one or more alkalizing agents (c) from the group consisting of potassium hydroxide, sodium hydroxide, sodium silicates, and potassium silicates in a total amount of from 0.1 to 2.5 wt.%, preferably from 0.15 to 1.5 wt.%, more preferably from 0.2 to 1.0 wt.% and particularly preferably from 0.25 to 0.5 wt.%, based on the total weight of the ready-to-use agent.

6. The agent according to one of claims 1 to 5, **characterized in that** it contains one or more compounds of formula (I) as ethoxylated fatty alcohol(s) (d) having a degree of ethoxylation of from 80 to 120,

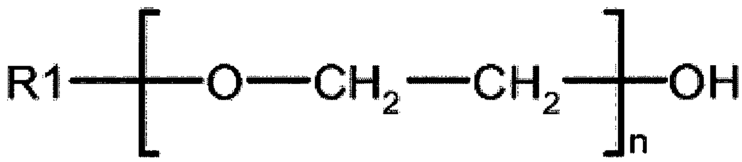

(I)

in which R1 stands for a saturated or unsaturated, unbranched or branched $C_8$ to $C_{24}$ alkyl group, preferably a saturated, unbranched $C_{16}$ or $C_{18}$ alkyl group, and
n stands for an integer from 80 to 120, preferably an integer from 90 to 110 and particularly preferably 100.

7. The agent according to one of claims 1 to 6, **characterized in that** it contains one or more ethoxylated fatty alcohols (d) having a degree of ethoxylation of from 80 to 120 in a total amount of from 0.1 to 1.5 wt.%, preferably from 0.2 to 1.2 wt.%, more preferably from 0.3 to 0.9 wt.% and particularly preferably from 0.4 to 0.8 wt.%, based on the total weight of the ready-to-use agent.

8. The agent according to one of claims 1 to 7, **characterized in that** it contains arachyl alcohol (eicosan-1-ol) and/or behenyl alcohol (docosan-1-ol) as the fatty alcohols (e).

9. The agent according to one of claims 1 to 8, **characterized in that** it contains the fatty alcohol(s) (e) from the group consisting of arachyl alcohol (eicosan-1-ol), gadoleyl alcohol ((9Z)-eicos-9-en-1-ol), arachidonyl alcohol ((5*Z*,8*Z*,11*Z*,14*Z*)-eicosa-5,8,11,14-tetraen-1-ol), behenyl alcohol (docosan-1-ol), erucyl alcohol ((13*Z*)-docos-13-en-1-ol) and brassidyl alcohol ((13*E*)-docosen-1-ol) in a total amount of from 0.7 to 5.0 wt.%, preferably from 0.9 to 4.0 wt.%, more preferably from 1.1 to 3.0 wt.% and particularly preferably from 1.2 to 2.8 wt.%, based on the total weight of the ready-to-use agent.

10. The agent according to one of claims 1 to 9, **characterized in that** it contains

(a) from 0.20 to 1.5 wt.% ammonia,
(b) from 0.2 to 6.5 wt.% monoethanolamine,
(c) from 0.1 to 2.5 wt.% potassium hydroxide,
(d) one or more ethoxylated fatty alcohols having a degree of ethoxylation of from 80 to 120,
and
(e) one or more fatty alcohols from the group consisting of arachyl alcohol (eicosan-1-ol), gadoleyl alcohol ((9*Z*)-eicos-9-en-1-ol), arachidonyl alcohol ((5*Z*,8*Z*,11*Z*,14*Z*)-eicosa-5,8,11,14-tetraen-1-ol), behenyl alcohol (docosan-1-ol), erucyl alcohol ((13*Z*)-docos-13-en-1-ol), and brassidyl alcohol ((13*E*)-docosen-1-ol), all the stated amounts being based on the total weight of the ready-to-use agent.

**11.** The agent according to one of claims 1 to 10, **characterized in that** it contains

(a) from 0.20 to 1.5 wt.% ammonia,
(b) from 0.2 to 6.5 wt.% monoethanolamine,
(c) from 0.1 to 2.5 wt.% sodium hydroxide,
(d) one or more ethoxylated fatty alcohols having a degree of ethoxylation of from 80 to 120, and
(e) one or more fatty alcohols from the group consisting of arachyl alcohol (eicosan-1-ol), gadoleyl alcohol ((9*Z*)-eicos-9-en-1-ol), arachidonyl alcohol ((5*Z*,8*Z*,11*Z*,14*Z*)-eicosa-5,8,11,14-tetraen-1-ol), behenyl alcohol (docosan-1-ol), erucyl alcohol ((13*Z*)-docos-13-en-1-ol), and brassidyl alcohol ((13*E*)-docosen-1-ol), all the stated amounts being based on the total weight of the ready-to-use agent.

**12.** The agent according to one of claims 1 to 11, **characterized in that** it contains

(a) ammonia,
(b) monoethanolamine,
(c) potassium hydroxide,
(d) one or more ethoxylated fatty alcohols having a degree of ethoxylation of from 80 to 120 that are selected from the group consisting of:

- octadecan-1-ol (octadecyl alcohol, stearyl alcohol) ethoxylated with 90 EO,
- octadecan-1-ol (octadecyl alcohol, stearyl alcohol) ethoxylated with 91 EO,
- octadecan-1-ol (octadecyl alcohol, stearyl alcohol) ethoxylated with 92 EO,
- octadecan-1-ol (octadecyl alcohol, stearyl alcohol) ethoxylated with 93 EO,
- octadecan-1-ol (octadecyl alcohol, stearyl alcohol) ethoxylated with 94 EO,
- octadecan-1-ol (octadecyl alcohol, stearyl alcohol) ethoxylated with 95 EO,
- octadecan-1-ol (octadecyl alcohol, stearyl alcohol) ethoxylated with 96 EO,
- octadecan-1-ol (octadecyl alcohol, stearyl alcohol) ethoxylated with 97 EO,
- octadecan-1-ol (octadecyl alcohol, stearyl alcohol) ethoxylated with 98 EO,
- octadecan-1-ol (octadecyl alcohol, stearyl alcohol) ethoxylated with 99 EO,
- octadecan-1-ol (octadecyl alcohol, stearyl alcohol) ethoxylated with 100 EO,
- octadecan-1-ol (octadecyl alcohol, stearyl alcohol) ethoxylated with 101 EO,
- octadecan-1-ol (octadecyl alcohol, stearyl alcohol) ethoxylated with 102 EO,
- octadecan-1-ol (octadecyl alcohol, stearyl alcohol) ethoxylated with 103 EO,
- octadecan-1-ol (octadecyl alcohol, stearyl alcohol) ethoxylated with 104 EO,
- octadecan-1-ol (octadecyl alcohol, stearyl alcohol) ethoxylated with 105 EO,
- octadecan-1-ol (octadecyl alcohol, stearyl alcohol) ethoxylated with 106 EO,
- octadecan-1-ol (octadecyl alcohol, stearyl alcohol) ethoxylated with 107 EO,
- octadecan-1-ol (octadecyl alcohol, stearyl alcohol) ethoxylated with 108 EO,
- octadecan-1-ol (octadecyl alcohol, stearyl alcohol) ethoxylated with 109 EO,
- octadecan-1-ol (octadecyl alcohol, stearyl alcohol) ethoxylated with 110 EO, and

(e) behenyl alcohol (docosan-1-ol).

**13.** The agent according to one of claims 1 to 12, **characterized in that** it contains

(a) ammonia,
(b) monoethanolamine,
(c) potassium hydroxide,
(d) one or more ethoxylated fatty alcohols having a degree of ethoxylation of from 80 to 120 that are selected from the group consisting of:

- octadecan-1-ol (octadecyl alcohol, stearyl alcohol) ethoxylated with 90 EO,
- octadecan-1-ol (octadecyl alcohol, stearyl alcohol) ethoxylated with 91 EO,
- octadecan-1-ol (octadecyl alcohol, stearyl alcohol) ethoxylated with 92 EO,
- octadecan-1-ol (octadecyl alcohol, stearyl alcohol) ethoxylated with 93 EO,
- octadecan-1-ol (octadecyl alcohol, stearyl alcohol) ethoxylated with 94 EO,
- octadecan-1-ol (octadecyl alcohol, stearyl alcohol) ethoxylated with 95 EO,
- octadecan-1-ol (octadecyl alcohol, stearyl alcohol) ethoxylated with 96 EO,

- octadecan-1-ol (octadecyl alcohol, stearyl alcohol) ethoxylated with 97 EO,
- octadecan-1-ol (octadecyl alcohol, stearyl alcohol) ethoxylated with 98 EO,
- octadecan-1-ol (octadecyl alcohol, stearyl alcohol) ethoxylated with 99 EO,
- octadecan-1-ol (octadecyl alcohol, stearyl alcohol) ethoxylated with 100 EO,
- octadecan-1-ol (octadecyl alcohol, stearyl alcohol) ethoxylated with 101 EO,
- octadecan-1-ol (octadecyl alcohol, stearyl alcohol) ethoxylated with 102 EO,
- octadecan-1-ol (octadecyl alcohol, stearyl alcohol) ethoxylated with 103 EO,
- octadecan-1-ol (octadecyl alcohol, stearyl alcohol) ethoxylated with 104 EO,
- octadecan-1-ol (octadecyl alcohol, stearyl alcohol) ethoxylated with 105 EO,
- octadecan-1-ol (octadecyl alcohol, stearyl alcohol) ethoxylated with 106 EO,
- octadecan-1-ol (octadecyl alcohol, stearyl alcohol) ethoxylated with 107 EO,
- octadecan-1-ol (octadecyl alcohol, stearyl alcohol) ethoxylated with 108 EO,
- octadecan-1-ol (octadecyl alcohol, stearyl alcohol) ethoxylated with 109 EO,
- octadecan-1-ol (octadecyl alcohol, stearyl alcohol) ethoxylated with 110 EO, and

(e) arachyl alcohol (eicosan-1-ol) and behenyl alcohol (docosan-1-ol).

**14.** A ready-to-use agent for dyeing and/or lightening keratinic fibers, **characterized in that** it is produced immediately before application by mixing preparations (A) and (B),

- preparation (A) being an agent of claims 1 to 13,
- preparation (B) being an agent that contains hydrogen peroxide in a cosmetic carrier, and
- the ready-to-use agent produced by mixing preparations (A) and (B) containing hydrogen peroxide in an amount of from 0.5 to 8.0 wt.%, preferably from 1.5 to 6.5 wt.%, more preferably from 2.2 to 4.5 wt.% and particularly preferably from 3.0 to 3.6 wt.% (calculated as 100% hydrogen peroxide), based on the total weight of the ready-to-use agent.

**Revendications**

**1.** Agent destiné à colorer et/ou à éclaircir des fibres kératiniques, notamment de cheveux humains, contenant dans un support cosmétique

(a) de l'ammoniaque,
(b) un ou plusieurs alcools aminés du groupe comprenant la monoéthanolamine, le 2-amino-2-méthyl-propanol et la triéthanolamine,
(c) un ou plusieurs agents alcalinisants du groupe comprenant l'hydroxyde de potassium, l'hydroxyde de sodium, les silicates de sodium et les silicates de potassium,
(d) un ou plusieurs alcools gras éthoxylés présentant un degré d'éthoxylation de 80 à 120,
(e) un ou plusieurs alcools gras du groupe comprenant l'alcool arachidylique (eicosan-1-ol), l'alcool gadoleyl ((9Z)-eicos-9-en-1-ol), l'alcool arachidonique ((5Z,8Z,11Z,14Z)-eicosa-5,8,11,14-tetraène-1-ol), l'alcool béhénylique (docosan-1-ol), l'alcool d'erucyl ((13Z)-docos-13-en-1-ol) et l'alcool brassidyl ((13E)-docosen-1-ol).

**2.** Agent selon la revendication 1 **caractérisé en ce qu'**il contient de l'ammoniaque (a) dans une quantité de 0,20 à 1,5 % en poids, de préférence de 0,25 à 1,2 % en poids, de manière davantage préférée de 0,30 à 1,0 % en poids et de manière particulièrement préférée de 0,31 à 0,8 % en poids, par rapport au poids total de l'agent prêt à l'emploi.

**3.** Agent selon une des revendications 1 ou 2, **caractérisé en ce qu'**il contient un ou plusieurs alcools aminés (b) du groupe comprenant la monoéthanolamine, le 2-amino-2-méthyl-propanol et la triéthanolamine dans une quantité totale de 0,2 à 6,5 % en poids, de préférence de 0,5 à 4,0 % en poids, de manière davantage préférée de 0,7 à 2,5 % en poids et de manière particulièrement préférée de 0,8 à 1,6 % en poids, par rapport au poids total de l'agent prêt à l'emploi.

**4.** Agent selon une des revendications 1 à 3 **caractérisé en ce qu'**il contient de l'hydroxyde de potassium et/ou de l'hydroxyde de sodium comme agent alcalinisant.

**5.** Agent selon une des revendications 1 à 4 **caractérisé en ce qu'**il contient un ou plusieurs agents alcalinisants (c) du groupe comprenant l'hydroxyde de potassium, l'hydroxyde de sodium, les silicates de sodium et les silicates de

potassium dans une quantité totale de 0,1 à 2,5 % en poids, de préférence de 0,15 à 1,5 % en poids, de manière davantage préférée de 0,2 à 1,0 % en poids et de manière particulièrement préférée de 0,25 à 0,5 % en poids, par rapport au poids total de l'agent prêt à l'emploi.

6. Agent selon une des revendications 1 à 5 **caractérisé en ce qu'**il contient, comme alcool(s) gras éthoxylé(s) (d) présentant un degré d'éthoxylation de 80 à 120 un ou plusieurs composés de formule (I),

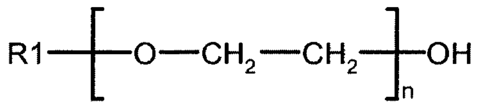

(I)

où R1 représente un groupe alkyle saturé ou insaturé, non ramifié ou ramifié en $C_8$-$C_{24}$, de préférence un groupe alkyle saturé, non ramifié en $C_{16}$ ou $C_{18}$, et
n représente un nombre entier de 80 à 120, de préférence un nombre entier de 90 à 110 et de manière particulièrement préférée le nombre 100.

7. Agent selon une des revendications 1 à 6 **caractérisé en ce qu'**il contient un ou plusieurs alcools gras éthoxylés (d) présentant un degré d'éthoxylation de 80 à 120 dans une quantité totale de 0,1 à 1,5 % en poids, de préférence de 0,2 à 1,2 % en poids, de manière davantage préférée de 0,3 à 0,9 % en poids et de manière particulièrement préférée de 0,4 à 0,8 % en poids, par rapport au poids total de l'agent prêt à l'emploi.

8. Agent selon une des revendications 1 à 7 **caractérisé en ce qu'**il contient de l'alcool arachidylique (eicosan-1-ol) et/ou de l'alcool béhénylique (docosan-1-ol) comme alcools gras (e).

9. Agent selon une des revendications 1 à 8 **caractérisé en ce qu'**il contient le ou les alcools gras (e) du groupe comprenant l'alcool arachidylique (eicosan-1-ol), l'alcool gadoleyl ((9Z)-eicos-9-en-1-ol), l'alcool arachidonique ((5Z,8Z,11Z,14Z)-eicosa-5,8,11,14-tetraène-1-ol), l'alcool béhénylique (docosan-1-ol), l'alcool d'erucyl ((13Z)-docos-13-en-1-ol) et l'alcool brassidyl ((13E)-docosen-1-ol) dans une quantité totale de 0,7 à 5,0% en poids, de préférence de 0,9 à 4,0 % en poids, de manière davantage préférée de 1,1 à 3,0 % en poids et de manière particulièrement préférée de 1,2 à 2,8 % en poids, par rapport au poids total de l'agent prêt à l'emploi.

10. Agent selon une des revendications 1 à 9 **caractérisé en ce qu'**il contient

(a) 0,20 à 1,5 % en poids d'ammoniaque,
(b) 0,2 à 6,5 % en poids de monoéthanolamine,
(c) 0,1 à 2,5 % en poids d'hydroxyde de potassium,
(d) un ou plusieurs alcools gras éthoxylés présentant un degré d'éthoxylation de 80 à 120 et
(e) un ou plusieurs alcools gras du groupe comprenant l'alcool arachidylique (eicosan-1-ol), l'alcool gadoleyl ((9Z)-eicos-9-en-1-ol), l'alcool arachidonique ((5Z,8Z,11Z,14Z)-eicosa-5,8,11,14-tetraène-1-ol), l'alcool béhénylique (docosan-1-ol), l'alcool d'erucyl ((13Z)-docos-13-en-1-ol) et l'alcool brassidyl ((13E)-docosen-1-ol), toutes les quantités indiquées se référant au poids total de l'agent prêt à l'emploi.

11. Agent selon une des revendications 1 à 10, **caractérisé en ce qu'**il contient

(a) 0,20 à 1,5 % en poids d'ammoniaque,
(b) 0,2 à 6,5 % en poids de monoéthanolamine,
(c) 0,1 à 2,5 % en poids d'hydroxyde de sodium,
(d) un ou plusieurs alcools gras éthoxylés présentant un degré d'éthoxylation de 80 à 120 et
(e) un ou plusieurs alcools gras du groupe comprenant l'alcool arachidylique (eicosan-1-ol), l'alcool gadoleyl ((9Z)-eicos-9-en-1-ol), l'alcool arachidonique ((5Z,8Z,11Z,14Z)-eicosa-5,8,11,14-tetraène-1-ol), l'alcool béhénylique (docosan-1-ol), l'alcool d'erucyl ((13Z)-docos-13-en-1-ol) et l'alcool brassidyl ((13E)-docosen-1-ol), toutes les quantités indiquées se référant au poids total de l'agent prêt à l'emploi.

12. Agent selon une des revendications 1 à 11 caractérisé en qu'il contient

(a) de l'ammoniaque,
(b) de la monoéthanolamine,
(c) de l'hydroxyde de potassium,
(d) un ou plusieurs alcools gras éthoxylés présentant un degré d'éthoxylation de 80 à 120, choisis dans le groupe comprenant

- l'octadécan-1-ol (alcool octadécylique, alcool stéarylique), éthoxylé avec 90 EO,
- l'octadécan-1-ol (alcool octadécylique, alcool stéarylique), éthoxylé avec 91 EO,
- l'octadécan-1-ol (alcool octadécylique, alcool stéarylique), éthoxylé avec 92 EO,
- l'octadécan-1-ol (alcool octadécylique, alcool stéarylique), éthoxylé avec 93 EO,
- l'octadécan-1-ol (alcool octadécylique, alcool stéarylique), éthoxylé avec 94 EO,
- l'octadécan-1-ol (alcool octadécylique, alcool stéarylique), éthoxylé avec 95 EO,
- l'octadécan-1-ol (alcool octadécylique, alcool stéarylique), éthoxylé avec 96 EO,
- l'octadécan-1-ol (alcool octadécylique, alcool stéarylique), éthoxylé avec 97 EO,
- l'octadécan-1-ol (alcool octadécylique, alcool stéarylique), éthoxylé avec 98 EO,
- l'octadécan-1-ol (alcool octadécylique, alcool stéarylique), éthoxylé avec 99 EO,
- l'octadécan-1-ol (alcool octadécylique, alcool stéarylique), éthoxylé avec 100 EO,
- l'octadécan-1-ol (alcool octadécylique, alcool stéarylique), éthoxylé avec 101 EO,
- l'octadécan-1-ol (alcool octadécylique, alcool stéarylique), éthoxylé avec 102 EO,
- l'octadécan-1-ol (alcool octadécylique, alcool stéarylique), éthoxylé avec 103 EO,
- l'octadécan-1-ol (alcool octadécylique, alcool stéarylique), éthoxylé avec 104 EO,
- l'octadécan-1-ol (alcool octadécylique, alcool stéarylique), éthoxylé avec 105 EO,
- l'octadécan-1-ol (alcool octadécylique, alcool stéarylique), éthoxylé avec 106 EO,
- l'octadécan-1-ol (alcool octadécylique, alcool stéarylique), éthoxylé avec 107 EO,
- l'octadécan-1-ol (alcool octadécylique, alcool stéarylique), éthoxylé avec 108 EO,
- l'octadécan-1-ol (alcool octadécylique, alcool stéarylique), éthoxylé avec 109 EO,
- l'octadécan-1-ol (alcool octadécylique, alcool stéarylique), éthoxylé avec 110 EO, et (e) de l'alcool béhénylique (docosan-1-ol).

**13.** Agent selon une des revendications 1 à 12 **caractérisé en ce qu'**il contient

(a) de l'ammoniaque,
(b) de la monoéthanolamine,
(c) de l'hydroxyde de potassium,
(d) un ou plusieurs alcools gras éthoxylés présentant un degré d'éthoxylation de 80 à 120, choisis dans le groupe comprenant

- l'octadécan-1-ol (alcool octadécylique, alcool stéarylique), éthoxylé avec 90 EO,
- l'octadécan-1-ol (alcool octadécylique, alcool stéarylique), éthoxylé avec 91 EO,
- l'octadécan-1-ol (alcool octadécylique, alcool stéarylique), éthoxylé avec 92 EO,
- l'octadécan-1-ol (alcool octadécylique, alcool stéarylique), éthoxylé avec 93 EO,
- l'octadécan-1-ol (alcool octadécylique, alcool stéarylique), éthoxylé avec 94 EO,
- l'octadécan-1-ol (alcool octadécylique, alcool stéarylique), éthoxylé avec 95 EO,
- l'octadécan-1-ol (alcool octadécylique, alcool stéarylique), éthoxylé avec 96 EO,
- l'octadécan-1-ol (alcool octadécylique, alcool stéarylique), éthoxylé avec 97 EO,
- l'octadécan-1-ol (alcool octadécylique, alcool stéarylique), éthoxylé avec 98 EO,
- l'octadécan-1-ol (alcool octadécylique, alcool stéarylique), éthoxylé avec 99 EO,
- l'octadécan-1-ol (alcool octadécylique, alcool stéarylique), éthoxylé avec 100 EO,
- l'octadécan-1-ol (alcool octadécylique, alcool stéarylique), éthoxylé avec 101 EO,
- l'octadécan-1-ol (alcool octadécylique, alcool stéarylique), éthoxylé avec 102 EO,
- l'octadécan-1-ol (alcool octadécylique, alcool stéarylique), éthoxylé avec 103 EO,
- l'octadécan-1-ol (alcool octadécylique, alcool stéarylique), éthoxylé avec 104 EO,
- l'octadécan-1-ol (alcool octadécylique, alcool stéarylique), éthoxylé avec 105 EO,
- l'octadécan-1-ol (alcool octadécylique, alcool stéarylique), éthoxylé avec 106 EO,
- l'octadécan-1-ol (alcool octadécylique, alcool stéarylique), éthoxylé avec 107 EO,
- l'octadécan-1-ol (alcool octadécylique, alcool stéarylique), éthoxylé avec 108 EO,
- l'octadécan-1-ol (alcool octadécylique, alcool stéarylique), éthoxylé avec 109 EO,
- l'octadécan-1-ol (alcool octadécylique, alcool stéarylique), éthoxylé avec 110 EO, et

(e) l'alcool arachidylique (eicosan-1-ol) et l'alcool béhénylique (docosan-1-ol).

**14.** Agent prêt à l'emploi destiné à colorer et/ou à éclaircir des fibres kératiniques, **caractérisé en ce qu'**il est fabriqué juste avant l'application par le mélange des préparations (A) et (B),

- la préparation (A) correspondant à un agent selon les revendications 1 à 13,
- la préparation (B) correspondant à un agent contenant du peroxyde d'hydrogène dans un support cosmétique, et
- l'agent prêt à l'emploi fabriqué par le mélange des préparations (A) et (B) comprenant du peroxyde d'hydrogène dans une quantité de 0,5 à 8,0 % en poids, de préférence de 1,5 à 6,5 % en poids, de manière davantage préférée de 2,2 à 4,5 % en poids et de manière particulièrement préférée de 3,0 à 3,6 % en poids (calculé comme du peroxyde d'hydrogène à 100 %), par rapport au poids total de l'agent prêt à l'emploi.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006060570 A
- WO 2006060565 A
- WO 2005110499 A
- JP 2007191459 B
- WO 2010015441 A1
- WO 2010066720 A2
- WO 2009043613 A1
- WO 2012079953 A2
- JP 2003040750 B